# EUROPEAN PATENT APPLICATION

(11) **EP 3 521 415 A1**
(43) Date of publication of application: **07.08.2019**
(21) Application number: 17856800.2
(22) Date of filing: 28.09.2017
(51) Int. Cl.: C12N 1/20, C12P 7/64, A61K 8/99, A61K 35/747, A61Q 19/00, A23L 33/135, A23K 10/16, C12R 1/225

(54) **NOVEL LACTOBACILLUS SAKEI AND COMPOSITION COMPRISING SAME**

(30) Priority: 30.09.2016 KR 20160126823
(71) Applicant: CJ Cheiljedang Corporation, Seoul 04560 (KR)
(72) Inventor: JEONG, Da Hye, Suwon-si Gyeonggi-do 16508 (KR); CHUNG, Young Mee, Yongin-si Gyeonggi-do 16846 (KR); KIM, Bong Joon, Yongin-si Gyeonggi-do 16951 (KR); KIM, So Young, Suwon-si Gyeonggi-do 16509 (KR); YUN, Hyun Sun, Seoul 06007 (KR); MOON, Byoung Seok, Anyang-si Gyeonggi-do 14105 (KR); PARK, Jie Eun, Anyang-si Gyeonggi-do 14061 (KR); BAE, Gi Duk, Daejeon 34636 (KR); AHN, Hee Yoon, Suwon-si Gyeonggi-do 04093 (KR); JANG, Jae Ho, Seoul 08000 (KR)
(74) Representative: Mewburn Ellis LLP
(86) International application number: PCT/KR2017/010873
(87) International publication number: WO 2018/062914

(57) **Abstract**

The present disclosure relates to a novel *Lactobacillus sakei* strain and a composition comprising the same.

## Description

### [Technical Field]

The present disclosure relates to novel *Lactobacillus sakei* and a composition comprising the same.

### [Background Art]

Recently, the population of those with obesity has significantly increased due to westernized diet and lifestyle. According to the report of National Health Insurance Service in 2013, the percentage of morbid obesity (BMI of 30 or higher) in Korea had increased 1.7 times since 2002, that is, from 2.5% (in 2002) to 4.2% (in 2013), and this trend has led to the prediction that by 2025, the percentage will increase to as high as 5.9%. Obesity is defined as a condition characterized by excessively increased adipose tissues due to greater energy intake than energy consumption, and may also be defined by indices such as BMI, *etc.* In Korea, obesity is defined as a BMI of 25 or higher, whereas in WHO and overseas, obesity is defined as a BMI of 30 or higher. Obesity has largely been highlighted in terms of cosmetic side effects; however, in addition to cosmetic aspects, increasing health concerns have recently drawn much attention to metabolic diseases caused by obesity, such as diabetes, hyperlipidemia, *etc.*

Interest in health, which has increased along with the term "well-being", has also greatly contributed to increases in various therapeutic foods and agents for obesity. Garcinia cambogia extracts (HCA), conjugated linoleic acid (CLA) (Korean Patent No. 10-1438177), *etc.,* which are anti-obesity health functional foods, are representatives thereof, and Orlistat (Zenical) is an FDA-approved drug. HCA and CLA are known to exhibit anti-obesity efficacy through the mechanisms of inhibiting lipogenesis from carbohydrates and reducing adipocytes, respectively. The FDA-approved drug Orlistat shows the same anti-obesity efficacy through inhibition of fat absorption; more precisely, it exhibits the anti-obesity effect by not absorbing 30% of the fat of the food intake into the intestines and excreting the same as feces.

These health functional foods and drugs have been used as various products in the market; however, side effects thereof are becoming an issue in various studies and new articles. The most widely known side effect is liver damage such as acute hepatitis, hepatic failure, *etc.* Although the causes have not yet been established, according to an announcement recently made by the National Evidence-based Healthcare Collaborating Agency (NECA), several domestic and foreign studies related to HCA disclosed that there were patients who suffered liver damage or cardiac disorders after ingesting health functional foods. Additionally, the U.S. FDA has recently prohibited sale of "hydrocut", a health functional food product containing HCA, and accordingly, more research on specific and detailed investigation of the causes are needed. However, more attention is required when selecting diet products when they contain such ingredients. There is currently an increasing demand for diet-related products, but due to safety issues thereof, various diet materials have been arising as alternative materials.

One of those materials is lactic acid bacteria. Lactic acid bacteria refer to bacteria having metabolic activity of degrading carbohydrates into lactic acids, and are known to be present mainly in fermented foods such as kimchi, cheese, yogurt, *etc.* (Korean Patent No. 10-1498229). The lactic acid bacteria are a Generally Recognized As Safe (GRAS) material, that is, they are acknowledged as a safe material according to the U.S. FDA, and their safety has been proved as they are present in many fermented foods that people usually ingest. The lactic acid bacteria, as their safety is guaranteed and they are naturally derived microorganisms, are more reliable than general synthetic chemical drugs or GMO foods. Several studies have found that with respect to liver damage, known to be a significant side effect of current diet products, lactic acid bacteria have a positive effect on non-alcoholic fatty liver or liver function.

In general, lactic acid bacteria have been commercialized in the form of dairy products, while highlighting their intestinal regulation function. Recently, the efficacy of lactic acid bacteria on immune enhancement and metabolic disease alleviation has been proved in addition to the intestinal regulation function, and thus, commercialization thereof into functional probiotics has been actively underway, and the sales are growing every year. Probiotics refer to microorganisms having useful functions, and lactic acid bacteria are a significant contributor thereto; the term "probiotics" suggests a more comprehensive meaning. The functions of immune enhancement and metabolic disease alleviation are explained in relation to gut microbiota, rather than being understood as direct functions of the lactic acid bacteria.

Studies on gut microbiota and obesity are increasing outstandingly every year, and those to prove anti-obesity efficacy using microbiota or single strain are underway. In addition, studies on the correlation between anti-obesity and short chain fatty acids (SFCA) of the gut microbiota, too, are currently underway.

### [Disclosure]

### [Technical Problem]

The present inventors endeavored to develop a strain which is more harmless to the human body and stable and has high anti-obesity efficacy, and as a result, they developed a *Lactobacillus sakei* CJLS03 strain capable of promoting production of a short chain fatty acid by controlling a gut microbiota.

### [Technical Solution]

An object of the present disclosure is to provide a *Lactobacillus sakei* CJLS03 strain (KCCM11905P).

Another object of the present disclosure is to provide a composition comprising the above strain.

### [Advantageous Effects]

According to the present disclosure, *Lactobacillus sakei* CJLS03 can promote the production of short chain fatty acids by adjusting a gut microbiota. More specifically, the *Lactobacillus sakei* CJLS03, by reducing the ratio of *Firmicutes*/*Bacteroidetes* (F/B) in the gut microbiota and increasing the amount of short chain fatty acids produced *in vivo,* accelerates oxidation and suppresses storage of internal fat, and thus is effective in terms of not only anti-obesity but also the prevention, improvement, or treatment of metabolic diseases. Further, the *Lactobacillus sakei* CJLS03 can suppress the fat accumulation in internal tissues and the increase of the size of adipocytes, and thus can be used as an ingredient having prophylactic, ameliorative, or therapeutic efficacy on obesity or metabolic diseases in various fields of drugs, feeds, cosmetics, foods, *etc.*

### [Brief Description of Drawings]

Fig. 1 is a graph showing the amounts of butyrate, acetate, and propionate measured after 3 hours of culturing of each group in an appendix sample according to Example 2.
Fig. 2 is a graph showing the amounts of butyrate, acetate, and propionate measured after 6 hours of culturing of each group in an appendix sample according to Example 2.
Fig. 3 is a graph showing the relative amount of *Firmicutes* measured after 6 hours of culturing of each group in an appendix sample according to Example 3.
Fig. 4 is a graph showing the relative amount of *Bacteroidetes* measured after 6 hours of culturing of each group in an appendix sample according to Example 3.
Fig. 5 is a graph showing the relative ratio of *Firmicutes*/*Bacteroidetes* measured after 6 hours of culturing of each group in an appendix sample according to Example 3.
Fig. 6 is a graph showing the relative amount of *Bifidobacterium* measured after 6 hours of culturing of each group in an appendix sample according to Example 3.
Fig. 7 is a graph showing change of the body weight of each group measured according to Example 4.
Fig. 8 is a graph showing the amount of feed intake of each group measured according to Example 4.
Fig. 9 is a graph showing the measurements of the blood components of each group according to Example 4.
Fig. 10 is a graph showing the weights of body and adipose tissues of each group according to Example 4.
Fig. 11 is a table showing relative weights of adipose tissues to body weight of each group according to Example 4.
Fig. 12 is an image of histological analysis of the liver tissue of ND, HFD, OLS, GC, and high CJLS03 groups.
Fig. 13 is a graph showing the sizes of the adipocytes of ND, HFD, OLS, GC, and high CJLS03 groups.
Fig. 14 is an image of adipocytes of ND, HFD, OLS, GC, and high CJLS03 groups.

### [Best Mode]

Hereinbelow, the present disclosure is described in more detail. Meanwhile, each description and exemplary embodiment disclosed in the present disclosure can be applied to other descriptions and exemplary embodiments. That is, all combinations of the various elements disclosed in the present disclosure fall within the scope of the present disclosure. Additionally, the scope of the present disclosure cannot be construed as being limited by the specific description below.

An aspect of the present disclosure for achieving the objects provides a *Lactobacillus sakei* CJLS03 strain (KCCM11905P).

As used herein, the term "*Lactobacillus*" is a microorganism of aerobic or facultative anaerobic gram positive bacillus widely distributed in nature. The microorganisms belonging to the genus *Lactobacillus* include *Lactobacillus sakei, etc.* The present inventors provide a novel strain belonging to *Lactobacillus sakei* under Accession No. KCCM11905P. This corresponds to a probiotic strain, and is harmless to the human body and can be used without side effects.

The *Lactobacillus sakei* CJLS03 has a 16s rRNA nucleotide sequence of SEQ ID NO: 1.

According to an exemplary embodiment, the CJLS03 strain may have activity of promoting intestinal production of short chain fatty acids, and/or controlling a gut microbiota.

As used herein, the term "short chain fatty acid (SCFA)" refers to a fatty acid having 6 or fewer carbons, and is also called a lower fatty acid. SCFAs are water-soluble, and are most abundant in milk fat. A significant portion of the SCFAs is absorbed in portal veins, which makes them distinctive from long chain fatty acids. Further, the SCFAs are the final metabolite of dietary fiber ingested by a host, and are known to be highly relevant to energy metabolism. The SCFAs can be an energy source of intestinal epithelial cells, and provide beneficial effects to health, such as enhancing the immune system, reducing inflammation, controlling energy metabolism, *etc.* For example, the ratio of acetate and propionate (A/P) among SCFAs in blood is closely related to BMI, and intestinal butyrate has an excellent therapeutic effect on metabolic diseases as it increases. More specifically, a dietary regimen having increased acetate or butyrate lowers incidence of diabetes and increases energy consumption.

The CJLS03 strain may have activity of promoting intestinal production of a SCFA. For example, the SCFA may be at least one selected from the group consisting of acetate, propionate, and butyrate. More specifically, the CJLS03 strain may increase intestinal production of at least one SCFA selected from the group consisting of acetate, propionate, and butyrate. That is, by increasing an *in vivo* amount of SCFAs, the CJLS03 strain can increase *in vivo* energy consumption, and can be useful in the treatment of metabolic diseases. For example, the CJLS03 strain can increase intestinal production of acetate, propionate, and/or butyrate by 1.5 to 2 times.

According to an exemplary embodiment, the CJLS03 strain may have activity of controlling a gut microbiota.

As used herein, the term "gut microbiota" refers to a microorganism microbiota present in the intestine, and is known that its number is as large as that of the cells in a human body and there are various types thereof. The gut microbiota influences not only the intestinal environment but also other organisms through metabolites or physical changes. For example, the intestinal microorganisms may affect change in production of a cytokine, which is a protein produced by *in vivo* immunocytes so as to change immune efficacy or reduce physical changes such as gut leakage and alleviate inflammation effects so as to increase immunity. With respect to metabolic diseases, the intestinal microorganisms may affect *in vivo* fat absorption, adipocyte production or reduction, *etc.,* or control of hormone secretion.

For example, gut microorganisms include *Firmicutes, Bacteroidetes, Proteobacteria, Actinobacteria, etc. Firmicutes* are gram positive, whereas *Bacteroidetes* are gram negative, and these are major occupiers of the gut microorganisms. Such microorganisms may influence obesity or metabolic diseases. For example, a study on the intestinal microorganisms and gut microbiota of an obese group and non-obese group has reported that the obese group includes a considerable amount of intestinal microorganisms of phylum *Firmicutes* whereas the non-obese group does not include many of intestinal microorganisms of phylum *Bacteroidetes.* This suggests that an increased level of intestinal *Firmicutes* may induce obesity as energy intake increases, whereas that of intestinal *Bacteroidetes* may suppress obesity.

The CJLS03 strain may have activity of controlling a gut microbiota. More specifically, the CJLS03 strain may have activity of reducing a ratio of intestinal *Firmicutes*/*Bacteroidetes* (F/B) compared to the case where no CJLS03 strain is included. For example, the CJLS03 strain may reduce the ratio of intestinal *Firmicutes*/*Bacteroidetes* to about 40% to 60%. In addition, the CJLS03 strain may increase the amounts of the microorganisms of genera *Lactobacillus* and *Bifidobacterium,* which are known as beneficial intestinal strains. In other words, the CJLS03 strain can reduce the ratio of intestinal *Firmicutes*/*Bacteroidetes* and increase beneficial intestinal strains, and thus can be useful in the prevention, improvement, or treatment of metabolic diseases.

Further, the CJLS03 strain has no *in vivo* side effects, and thus can suppress fat accumulation in tissues and increase in adipocyte size. The CJLS03 strain can exhibit an anti-obesity efficacy similar to or more excellent than anti-obesity materials known in the art, although it may vary depending on the amount of CJLS03 intake or the subject. More specifically, the CJLS03 strain can inhibit the fat accumulation in a mesenteric or subcutaneous adipose tissue and the increase of the adipocyte size.

The CJLS03 strain may have activity of reducing an aspartate transaminase (AST; GOT) level or an alanine transaminase (ALT; GPT) level. When a high fat food or an alcoholic beverage is continuously consumed, the liver functions may be partially damaged, which may induce a liver disease such as fatty liver. Conventionally, the liver test is conducted via an AST or ALT blood test, in which the AST or ALT level of 40 IU/L or below is diagnosed as normal and a higher level is diagnosed as having liver damage. Some of the conventionally known anti-obesity materials are known to be able to inhibit fat accumulation in an adipose tissue, but cannot reduce the AST or ALT level. In other words, since some of the conventionally known anti-obesity materials cannot prevent or ameliorate liver tissue damage, the risk of a liver disease such as fatty liver may still remain even if the materials are consumed. However, the CJLS03 strain, compared to the case where no CJLS03 strain is included, can inhibit an increase of *in vivo* AST and/or ALT level or reduce the *in vivo* AST and/or ALT level. Contrary to some known anti-obesity materials, the CJLS03 strain not only prevents fat accumulation in adipose tissue but also inhibits fat accumulation in a liver tissue and prevents or treats liver tissue damage.

Another aspect of the present disclosure provides a composition including the CJLS03 strain. The detailed description regarding the strain is as previously described.

The composition may further include a cryoprotectant or an excipient. More specifically, the composition may further include at least one cryoprotectant selected from the group consisting of glycerol, trehalose, maltodextrin, powdered skim milk, and starch; and/or at least one excipient selected from the group consisting of glucose, dextrin, and nonfat milk. The cryoprotectant of the present disclosure may be included in an amount of 0.01 wt% to 20 wt% or 0.01 wt% to 10 wt% based on the total weight of the composition. Specifically, the glycerol, trehalose, maltodextrin, powdered skim milk, and starch may be included in the composition in an amount of 5 wt% to 20 wt%, 2 wt% to 10 wt%, 2 wt% to 10 wt%, 0.5 wt% to 2 wt%, and 0.1 wt% to 1 wt%, respectively. Further, the excipient may be included in an amount of 75 wt% to 95 wt% or 85 wt% to 95 wt% based on the total weight of the composition.

According to an exemplary embodiment, the composition may be one selected from the group consisting of a food, a functional food, a feed, a feed additive, a cosmetic composition, a pharmaceutical composition, and a quasi-drug.

Still another aspect of the present disclosure provides a composition for prevention, improvement, or treatment of a metabolic disease, comprising the CJLS03 strain.

As used herein, the term "metabolic disease" collectively refers to all types of diseases that occur due to metabolic problems, and is also called metabolic disorder or metabolic syndrome. The metabolic disease includes without limitation the disease that can be prevented or treated using the *Lactobacillus sakei* CJLS03 strain as an active ingredient; for example, the metabolic disease can be at least one selected from the group consisting of obesity, diabetes, hypertension, hyperlipidemia, arteriosclerosis, stroke, and cardiovascular disease.

As used herein, the term "diabetes" refers to diseases that occur when there is deficient insulin secretion or insufficient action or function of insulin. Diabetes causes abnormal elevation of glucose concentration in blood due to excessive degradation of protein and lipids, which may result in glycosuria and ketonuria. Diabetes may also cause metabolic disorders such as hemoconcentration, circulatory disturbance, or renal disorders, which are induced by loss of electrolytes caused by metabolic abnormality of moisture and electrolytes. Diabetes can be classified into insulin-dependent diabetes mellitus (TypeI) and insulin-independent diabetes mellitus (Type II). Insulin is a substance secreted from β-cells of islet of Langerhans present inside the pancreas, and to regulate metabolic activities of energy sources, secretion of insulin is accelerated when the blood glucose concentration increases, whereas it is suppressed when the blood glucose concentration decreases. Diabetes is generally diagnosed by measuring the concentration of glucose in blood. For example, humans are diagnosed to have diabetes when normal glucose concentration in blood is 200 mg/dL or higher or when fasting glucose concentration in blood is 140 mg/dL or higher. Accordingly, diabetes can be treated or prevented if the glucose concentration is reduced in the blood or the liver.

As used herein, the term "hypertension" refers to a condition where the pressure of the arteries is chronically high. Hypertension also refers to a case when an adult of the age of 18 or higher has a systolic pressure of 140 mmHg or higher or a diastolic pressure of 90 mmHg or higher, and may occur due to obesity, *etc.*

As used herein, the term "hyperlipidemia" refers to a disease that occurs due to an elevated level of lipids in blood due to abnormal fat metabolism of triglycerides, cholesterols, *etc.* Specifically, hyperlipidemia includes hypercholesterolemia or hypertriglyceridemia, which is a condition having elevated levels of lipid components such as triglycerides, LDL cholesterol, phospholipids, free fatty acids, *etc.*

As used herein, the term "arteriosclerosis" refers to a condition where fatty plaques containing cholesterol, phospholipids, calcium, *etc.* are accumulated underneath the intima of the blood vessels, causing the arteries to harden and lose elasticity, thereby leading to suppression of blood supply and elevation of blood pressure, and eventually arteriorrhexis, artery dissection, *etc.*

As used herein, the term "stroke" refers to a disease caused by an abrupt impairment of part or the entirety of brain function lasting for a considerable period of time. Stroke includes cerebral infarction (ischemic stroke) that occurs as a result of an obstruction within brain blood vessels, and cerebral hemorrhage (hemorrhagic stroke) that occurs when blood vessels rupture, spilling blood into brain tissues.

As used herein, the term "cardiovascular disorder" refers to a disease that involves the heart and major blood vessels. Major cardiovascular disorder diseases include hypertension, ischemic heart disease, coronary heart disease, angina, myocardial infarction, atherosclerosis (arteriosclerosis), cerebrovascular disease, stroke, and arrhythmia.

The strain of the present disclosure has therapeutic and prophylactic effects on the obesity, diabetes, hypertension, hyperlipidemia, arteriosclerosis, stoke, and cardiovascular disorders described above.

As used herein, the term "prevention" refers to any behavior resulting in suppression or delay of the onset of metabolic diseases such as obesity, diabetes, hypertension, hyperlipidemia, arteriosclerosis, stoke, cardiovascular disorders, *etc.* by administering the composition according to the present disclosure, and the term "alleviation" or "treatment" refers to any action resulting in alleviation of symptoms of said diseases or beneficial alteration thereof by administering the composition according to the present disclosure.

The composition for prevention, improvement, or treatment of the metabolic diseases may be a drug composition which can be used as a pharmaceutical composition. If the composition of the present disclosure is a pharmaceutical composition, the composition may include a pharmaceutically acceptable carrier.

The composition comprising a pharmaceutically acceptable carrier may be in various oral or parenteral formulations. When formulating the composition, commonly used diluents or excipients, such as fillers, extenders, binders, wetting agents, disintegrants, surfactants, *etc.* can be used. Solid formulations for oral administration include tablets, pills, powders, granules, capsules, *etc.,* and such solid formulations are prepared by mixing at least one excipient *(e.g.,* starch, calcium carbonate, sucrose or lactose, gelatin, *etc*.) with at least one compound. In addition to simple excipients, lubricants such as magnesium stearate or talc are also used. Liquid formulations for oral administration include suspensions, solutions, emulsions, and syrup, *etc.,* and in addition to water and liquid paraffin, which are frequently used simple diluents, they may contain various excipients such as wetting agents, flavoring agents, aromatics, and preservatives. Formulations for parenteral administration include sterilized aqueous solutions, non-aqueous solvents, suspensions, emulsions, cryoprotectants, and suppositories. As non-aqueous solvents or suspending agents, propylene glycol, polyethylene glycol, plant oils such as olive oil, injectable esters such as ethyl oleate, *etc.* can be used.

The pharmaceutical composition may have one formulation selected from the group consisting of tablets, pills, powders, granules, capsules, suspensions, solutions, emulsions, and syrup, sterilized aqueous solutions, non-aqueous solvents, suspensions, emulsions, cryoprotectants, and suppositories.

According to an exemplary embodiment, the composition may further comprise a cryoprotectant or an excipient. More specifically, the composition may further comprise at least one cryoprotectant selected from the group consisting of glycerol, trehalose, maltodextrin, powdered skim milk, and starch. The cryoprotectant of the present disclosure may be contained in an amount of 0.01 wt% to 20 wt% or 0.01 wt% to 10 wt% based on the total weight of the composition; specifically, the glycerol may be contained in an amount of 5 wt% to 20 wt%, the trehalose may be contained in an amount of 2 wt% to 10 wt%, the maltodextrin may be contained in an amount of 2 wt% to 10 wt% while the powdered skim milk may be contained in an amount of 0.5 wt% to 2 wt%, and the starch may be contained in an amount of 0.1 wt% to 1 wt% in the composition based on the total weight of the composition. Further, the excipient may be at least one selected from the group consisting of glucose, dextrin, and nonfat milk. More specifically, the excipient may be contained in an amount of 75 wt% to 95 wt% or 85 wt% to 95 wt% based on the total weight of the composition.

The composition of the present disclosure is administered in a pharmaceutically effective amount. As used herein, the term "pharmaceutically effective amount" refers to an amount sufficient to treat or prevent a disease at a reasonable risk/benefit ratio applicable to any medical treatment. An effective dosage level may be determined depending on factors including the type of a subject and severity of the disease, age, gender, activity of the drug, subject's sensitivity to the drug, administration time, route of administration, excretion rate, duration of treatment, drugs used in combination, and other factors well known in the medical field.

The prophylactic or therapeutic agent of the present disclosure for the metabolic diseases, e.g., obesity, diabetes, hypertension, hyperlipidemia, arteriosclerosis, stoke, cardiovascular disorders, *etc.,* can be administered daily or intermittently. A daily dose can be administered at once or divided into two to three administrations. If two active ingredients are single formulations, the number of administration thereof may be the same or different. Additionally, the composition of the present disclosure can be used alone or in combination with other drugs for the prevention or treatment of liver diseases. It is important to administer the composition in a minimum amount which can exhibit a maximum effect without causing side effects in consideration of all factors described above, and this amount can be easily determined by one of ordinary skill in the art.

As another aspect, the present disclosure provides a method for preventing, improving, or treating a metabolic disease, comprising administering the composition to a subject in need thereof.

As used herein, the term "subject" may refer to all animals including humans in which a cancer has been developed or is likely to be developed. The diseases can be effectively prevented and treated by administering the composition of the present disclosure including the *Lactobacillus sakei* CJLS03 strain (KCCM11905P) as an active ingredient to the subject. The subject refers to all mammals including a dog, cow, horse, rabbit, mouse, rat, chicken, or human, but is not limited thereto.

One of ordinary skill in the art can understand that an optimal dosage and dosing interval for an individual administration of the composition of the present disclosure will be determined depending on the nature and extent of the disease being treated, the dosage form, the route and site of administration, and the age and physical condition of the subject being treated. It is possible for such administration to be repeated frequently as appropriate. If there is any side effect, dosage and frequency can be changed or reduced according to normal clinical treatment.

Any conventional route of administration can be used for the composition as long as the composition reaches target tissues. The composition of the present disclosure can be administered intraperitoneally, intravenously, intracutaneously, or orally, but is not limited thereto. Further, the composition can be administered by any device which enables the active substance to move to a target cell.

Still another aspect of the present disclosure provides a food composition comprising the strain.

Specifically, the composition of the present disclosure can be added to a food composition for the prevention or alleviation of metabolic diseases such as obesity, diabetes, hypertension, hyperlipidemia, arteriosclerosis, stroke, cardiovascular disorders, *etc.* The *Lactobacillus sakei* CJLS03 strain (KCCM11905P) is the same as described above. The food composition may comprise a sitologically acceptable carrier.

The food composition of the present disclosure includes all forms of a functional food, a nutritional supplement, a health food, a food additive, *etc.,* and such food compositions can be manufactured in various forms according to conventional methods known in the art.

When the *Lactobacillus sakei* CJLS03 strain is used as a food additive, the strain can be added as it is or it can be used with other foods or food ingredients, and can be used appropriately according to conventional methods. Amounts of active ingredients being mixed can be appropriately determined according to purpose of use (prevention, health, or therapeutic treatment). When preparing a food or drink, the active ingredients are generally added to a raw composition comprising a *Lactobacillus sakei* CJLS03 strain in an amount of 0.0001 wt% to 1 wt%, specifically 0.001 wt% to 0.1 wt%. In the case of long-term intake for health and hygiene or for health control, however, the amount can be less.

The type of the foods is not particularly limited. Examples of the foods that can be added by the substances include meat, sausages, bread, chocolate, candies, snacks, confectionery, pizzas, ramen noodles, other noodles, gums, dairy products including ice creams, various soups, drinks, teas, energy drinks, alcoholic drinks, and multi-vitamins. The foods include all health foods in a conventional sense.

The health drink composition of the present disclosure, as conventional drinks, may contain various flavoring agents, natural carbohydrates, *etc.* as additional ingredients. The previously described natural carbohydrates are monosaccharides (*e.g*., glucose and fructose), disaccharides *(e.g.,* maltose and sucrose), polysaccharides (*e.g.,* dextrin and cyclodextrin), and sugar alcohols *(e.g.,* xylitol, sorbitol, erythritol, *etc*.). As sweeteners, natural sweeteners (*e.g*., thaumatin and stevia extract), synthetic sweeteners (*e.g.,* saccharin and aspartame), *etc.* may be included. The ratio of the additional ingredients may be 0.01 parts by weight to 0.04 parts by weight, specifically 0.02 parts by weight to 0.03 parts by weight based on 100 parts by weight of the composition of the present disclosure.

In addition to the above, the composition of the present disclosure may contain various nutrients, vitamins, electrolytes, flavoring agents, coloring agents, pectic acids and salts thereof, alginic acids and salts thereof, organic acids, protective colloid thickening agents, pH-adjusting agents, stabilizers, preservatives, glycerin, alcohols, carbonating agents used in carbonated drinks, *etc.* The ratios of the additives are not significant, but the additives are generally selected in the range of 0.01 parts by weight to 0.1 parts by weight based on 100 parts by weight of the composition of the present disclosure. Further, the composition of the present disclosure may contain flesh for the manufacture of natural fruit juices, fruit juice, and vegetable drinks. The ratio of the flesh is not significant, but the flesh is generally selected in the range of 0.01 parts by weight to 10 parts by weight based on 100 parts by weight of the composition of the present disclosure. These ingredients can be used independently or in combination.

Still another aspect of the present disclosure provides a feed or feed additive composition comprising the strain.

The *Lactobacillus sakei* CJLS03 strain (KCCM11905P) is the same as previously described. Specifically, the *Lactobacillus sakei* CJLS03 strain of the present disclosure can be added to a feed additive for the prevention or amelioration of a metabolic disease or a feed composition comprising the same.

As used herein, the term "feed additive" refers to a substance added to a feed for various purposes such as supplementation of nutrients and prevention of body weight reduction, improvement of digestion utilizability of cellulose in the feed, improvement of milk quality, prevention of reproductive failure and enhancement of conception rate, prevention of high-temperature stress in summer, *etc.* The feed additive of the present disclosure corresponds to a supplementary feed according to the Control of Livestock and Fish Feed Act, minerals such as sodium carbohydrates, bentonite, magnesium oxide, composite minerals, *etc.;* trace minerals such as zinc, copper, cobalt, selenium, *etc.;* vitamins such as carotene, vitamin E, vitamins A, D, and E, nicotinic acid, vitamin B complex, *etc.;* amino acid protective agents such as methionine, lysine, *etc.;* fatty acid protective agents such as fatty acid calcium, *etc.;* live bacterial cell and yeast such as probiotics (lactic acid bacteria), yeast cultures, fungus cultures, *etc.*

As used herein, the term "feed" is any appropriate natural or artificial diet, single meal, *etc.,* for animals to eat, ingest, and digest, or an element of the single meal. A feed comprising the composition of the present disclosure for preventing or treating metabolic diseases can be manufactured in various feed forms known in the art, specifically including concentrated feeds, roughage, and/or special feeds.

The concentrated feeds include seed fruits including grains such as wheat, oats, corn, *etc.;* bran such as rice bran, wheat bran, barley bran, *etc.,* which are byproducts from refining grains; dregs, which are byproducts from extracting oil from soybeans, canola, sesame, flaxseed, coconut palm, *etc.;* residue such as residual starch substances, which are the main component of starch residue that remains after removing starch from sweet potatoes, potatoes, *etc.;* fish soluble which is a concentrate of fresh liquid obtained from fish meal, fish waste, and fish; animal-based feed such as meat meal, blood meal, feather meal, powdered skim milk, dried whey, obtained from drying whey, which is a residue from producing cheese from milk or casein from skim milk, *etc.;* yeasts, chlorella, or seaweeds, but the concentrated feeds are not limited thereto.

The roughage may include fresh grass feed such as wild grass, herbage, green manure, *etc.;* root vegetables such as turnips for feed, beets for feed, rutabagas, which are a type of turnip, *etc.;* silage, which is a storage feed obtained by placing fresh grass, green manure, paper mulberries, *etc.,* in a silo and performing lactic acid fermentation; dried grass obtained by cutting and drying wild grass and herbage, straw of crops for breeding stock; and leaves of beans, but is not limited thereto. The special feed may include mineral feed such as oyster shells, halite, *etc.;* urea feed such as urea or its derivatives, diureide isobutene, *etc.;* and feed additives or dietary supplements, which are added in small amounts to a mixed feed in order to supplement ingredients which may be lacking when only mixing natural feed ingredients or to increase the shelf life of feed, but is not limited thereto.

The feed composition of the present disclosure for preventing or improving the metabolic diseases can be prepared according to various feed production methods known in the art by adding the *Lactobacillus sakei* CJLS03 strain in an appropriate range of effective concentration.

The subject to which the feed composition of the present disclosure can be applied is not particularly limited as long as it targets prevention, improvement, or treatment of metabolic diseases. For example, the feed composition can be applied to any subject; that is, non-human animals such as monkeys, dogs, cats, rabbits, guinea pigs, rats, mice, cows, sheep, pigs, goats, *etc.;* birds; fishes; *etc.*

Still another aspect of the present disclosure provides a cosmetic composition comprising the strain.

The cosmetic composition may be manufactured in a formulation selected from the group consisting of an aqueous solution, topical ointments, creams, foams, nutritive cosmetic water, softening cosmetic water, facial masks, softening water, milky liquid, make-up base, essence, soap, an aqueous cleaning solution, bathing soap, sunscreen cream, sun oils, suspensions, emulsions, pastes, gels, lotions, powders, soaps, surfactant-containing cleansing, oil, powder foundation, emulsion foundation, wax foundation, patch, and spray, but is not limited thereto. When applied to a skin, the cosmetic composition of the present disclosure can provide excellent tactility and may include formulations directly applicable to skins such as topical ointment, cream, milky liquid, make-up base, soap, UV protection cream, paste, gel, lotion, suspension foundation, wax foundation, lip balm, *etc.,* but is not limited thereto.

In addition, the cosmetic composition of the present disclosure may further include at least one cosmetically acceptable carrier which is formulated with general skin cosmetics, for example, oil, water, surfactants, humectants, lower alcohols, thickening agents, chelating agents, dyes, preservatives, and fragrances, but is not limited thereto. The cosmetically acceptable carrier contained in the cosmetic composition of the present disclosure may be variously selected according to the form of the composition. For example, when the formulation is an ointment, paste, cream, or gel, it is not limited, but animal oils, vegetable oils, waxes, paraffin, starch, tragacanth, cellulose derivatives, polyethylene glycols, silicones, bentonites, silica, talc, zinc oxide, or a mixture thereof may be used as a carrier component.

The cosmetic composition of the present disclosure can be formulated into a functional cosmetic product by further comprising an ingredient having anti-oxidant, wrinkle-improving, anti-aging, and/or sun protection effects. For the ingredient having anti-oxidant, wrinkle-improving, anti-aging, and/or UV protection effects, any active ingredients used in functional cosmetic products known in the art can be used as ingredients having the anti-oxidant, wrinkle-improving, anti-aging, and/or UV protection effects without limitation.

Still another aspect of the present disclosure provides a quasi-drug comprising the strain.

As used herein, the term "quasi-drugs" refer to, among articles used for the diagnosis, treatment, amelioration, alleviation, healing, or prevention of diseases of humans or animals, those having milder action than drugs; for example, quasi-drugs according to the pharmaceutical laws refer to those excluding articles for medical use, and include products used for treatment or prevention of diseases of humans or animals, those having mild or indirect action on the human body, *etc.*

The quasi-drug composition of the present disclosure may be prepared in the formulation selected from the group consisting of body cleanser, foam, soap, facial mask, ointment, cream, lotion, essence, and spray, but is not limited thereto.

When the composition of the present disclosure is used as an additive for quasi-drugs, the composition can be added alone or used in combination with other quasi-drugs or quasi-drug ingredients. The composition can be appropriately used according to conventional methods. Amounts of active ingredients being mixed can be appropriately determined according to purpose of use.

### [Mode for Invention]

Hereinafter, the present disclosure will be described in more detail with reference to the following exemplary embodiments. However, these exemplary embodiments are for illustrative purposes only, and the present disclosure is not intended to be limited by these exemplary embodiments.

### Example 1: Isolation and identification of Lactobacillus sakei CJLS03 strain

The CJLS03 of the present disclosure, a novel *Lactobacillus sakei* strain isolated from kimchi, a traditional Korean food, was chosen to be a probiotics strain candidate through basic property evaluation and safety inspection. The *Lactobacillus sakei* CJLS03 was mixed with 20% glycerol and stored in a -80°C quick freezer. When used for an experiment, the strain was cultured for 18 hours to 48 hours in an MRS solid or liquid medium.

The 16S rRNA nucleotide sequence of the *Lactobacillus sakei* CJLS03 of the present disclosure was examined for identification and classification of the microorganisms, and as a result, the strain showed the highest homology (99.9%) to a standard *Lactobacillus sakei* strain (*Lactobacillus sakei* gene for 16S ribosomal RNA, partial sequence, strain: qz1215) and the highest molecular phylogenetic relationship.

Consequently, the novel microorganism chosen via the above process was identified using *Lactobacillus sakei,* and was named *Lactobacillus sakei* CJLS03. The *Lactobacillus sakei* CJLS03 was deposited on September 26, 2016, at the Korean Culture Center of Microorganisms (KCCM), an international depositary institution under the Budapest Treaty, and received Accession No. KCCM11905P. The nucleotide sequence of 16S rRNA gene of *Lactobacillus sakei* CJLS03 is the same as SEQ ID NO. 1 of the sequence listing attached hereto.

The *Lactobacillus sakei* CJLS03 of the present disclosure is gram positive, and is a facultative anaerobe as it can grow under both aerobic and anaerobic conditions. The novel bacteria do not from a spore or move, and have a rod shape. More specific morphological and physiological properties of the *Lactobacillus sakei* CJLS03 were analyzed using well-known methods in the art, and the results thereof are shown in Table 1 below. The ability of the strain to utilize sugars was determined using API50CH and API50CHL kits (Biomeriuex). The consequently confirmed morphology and physiological and biochemical properties of the CJLS03 were summarized in Table 1 below.

**[Table 1]**

| **Morphological, physiological, and biochemical properties** | **Result** |
|---|---|
| Morphology | *Bacillus* (rod) |
| Motility | - |
| Spore | - |
| Catalase | - |
| Homo-hetero fermentation | Facultative hetero fermentation |
| Proliferation at 15°C | + |
| Proliferation at 45°C | - |
| Proliferation at 3% NaCl | + |
| Anaerobic proliferation | + |
| CO₂ production using glucose | - |

| **Properties of Sugar fermentation** | |
|---|---|
| Glycerol | - |
| Erythritol | - |
| D-Arabinose | - |
| L-Arabinose | + |
| Ribose | + |
| D-Xylose | - |
| L-Xylose | - |
| Adonitol | - |
| Xyloside | - |
| Galactose | + |
| D-Glucose | + |
| D-Fructose | + |
| D-Mannose | + |
| L-Sorbose | - |
| Rhamnose | - |
| Dulcitol | - |
| Inositol | - |
| Mannitol | - |
| Sorbitol | - |
| D-Mannoside | - |
| D-Glucoside | - |
| Glucosamine | + |
| Amygdalin | - |
| Albutin | - |
| Esculin | + |
| Salicin | - |
| Cellobiose | + |
| Maltose | + |
| Lactose | + |
| Melibiose | + |
| Saccharose | + |
| Trehalose | - |
| Inulin | - |
| Melizitose | - |
| D-Raffinose | - |
| Amidon | - |
| Glycogen | - |
| Xylitol | - |
| Gentiobiose | - |
| D-Turanose | - |
| D-Lyxose | - |
| D-tagatose | - |
| D-Fucose | - |
| L-Fucose | - |
| D-Arabitol | - |
| L-Arabitol | - |
| Gluconate | + |
| 2-Gluconate | - |
| 5-Gluconate | - |

| | |
|---|---|
| +: positive reaction -: negative reaction | |

### Example 2: Experimentation of confirming SCFA via in vitro fermentation

*In vitro* fermentation is a simple comparative experimentation method, which involves co-culturing each sample of feces/appendix of a normal-diet mouse with lactic acid bacteria and measuring changes in the amount of short chain fatty acids to investigate intestinal productivity of short chain fatty acids, and the *in vitro* fermentation using feces samples is a known method in the art (Yang *et al*., 2013, Salazar *et al*., 2009, Hughes *et al*., 2008). However, the *in vitro* fermentation method has a problem in that when the remaining feces, which were not absorbed in the intestines, were used as a substrate for fermentation by the lactic acid bacteria, the amount of substrate is not sufficient, leading to improper fermentation. Accordingly, in this experimentation, a known method in which the contents of pig appendix were used to study intestinal metabolic substances (Labib *et al*., 2004) was used to perform *in vitro* fermentation to which the appendix contents having a larger amount of substrates than feces.

Specifically, the appendix contents of the normal-diet mouse not ntreated with antibiotics and probiotics were put into an anaerobic pack (MGC) immediately after they were collected, and then stored at -80°C before initiation of the experimentation (Yang *et al*., 2013). During the experimentation, the stored sample was melted in an anaerobic chamber (Coy laboratory), to which 1:2 (w/v) of sterilized PBS was later added, and homogenized for 2 minutes. The experiment and control groups used for this experiment are as shown in Table 2 below.

**[Table 2]**

| # | **Strains** | **Key feature** |
|---|---|---|
| 1 | PBS | Negative control |
| 2 | CJLS03 | The strain of the present disclosure |
| 3 | OK101 | Granted anti-obesity strain (*L. sakei*) |
| 4 | LGG | Probiotics strain (*L. rhamnosus*) |

In order to analyze short chain fatty acids, a known SCFA analysis method was used, the specific method of which is as follows: volatile fatty acid mixture (ultrapure; Sigma (Supelco)) was used to analyze the standard curve and the retention time of peak detection, short chain fatty acids were extracted from feces and blood and then compared with the standard curve for analysis. The extraction of short chain fatty acids was performed according to an extraction method known in the art (Schwiertz *et al.,* 2010). An extract including 0.1 mol/L oxalic acid and 40 mmol/L sodium azide was mixed with the sample (max. 80 mg), and shake incubated for one hour at room temperature followed by separating the supernatant using centrifugation. The supernatant obtained by the centrifugation was analyzed using GC (Shimadzu GC2010) and a flame ionized detector (FID). The column used is HP INNO-WAS 30 m × 32 mm with a splitter temperature of 260°C and a FID of 260°C, and the column was analyzed at a temperature of 100°C to 180°C, 25°C/m, and 27.1 psi.

In this Example, 5 × 10⁸ CFU of each of the strains according to Table 2 was inoculated in homogenized appendix samples, and shake incubated at 37°C for 24 hours at maximum in an anaerobic environment. During the incubation, sampling of the culture was carried out at hours 3 and 6 from each group, and the SCFA of each group was analyzed according to the SCFA analysis method.

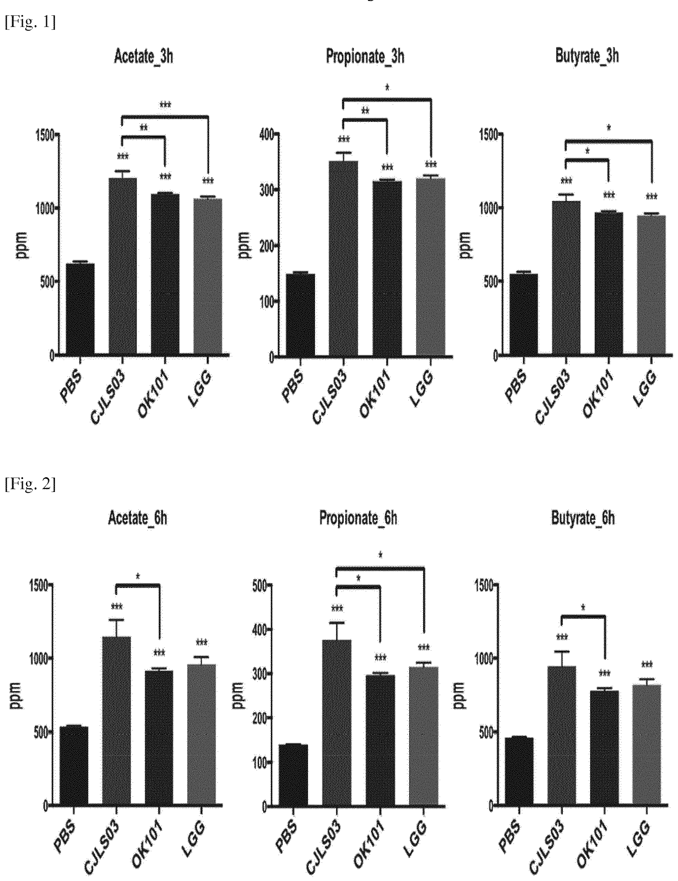

Fig. 1 shows the amounts of butyrate, acetate, and propionate, which were measured in the appendix sample after 3 hours of incubation, and Fig. 2 shows the amounts of butyrate, acetate, and propionate, which were measured in the appendix sample after 6 hours of incubation.

Based on Figs. 1 and 2, the amounts of short chain fatty acids (butyrate, acetate, and propionate) produced by culturing the *Lactobacillus sakei* CJLS03 of the present disclosure were greater than those known to be produced by culturing OK101 or LGG, a conventionally known *Lactobacillus sakei* strain. Further, in comparison of Figs. 1 and 2, the difference in the short chain fatty acid amounts was greater after 6 hours of incubation compared to 3 hours of incubation.

That is, the *Lactobacillus sakei* CJLS03 according to the present disclosure promotes the production of short chain fatty acids in the intestines such as appendix, thereby increasing the amount of short chain fatty acids.

### Example 3: Experiment on changes of microbiota via in vitro fermentation

According to the studies related to the correlation between metabolic diseases and gut microbiota, relative amounts of *Bacteroidetes* and *Firmicutes* are highly related to the metabolic diseases. In particular, the relative amount of the intestinal *Firmicutes* has been reported to be proportional to obesity or metabolic diseases.

In this Example, the microorganisms generated in an appendix sample, to which lactic acid bacteria are applied, were analyzed.

Specifically, the appendix contents of the normal-diet mouse not treated with antibiotics and probiotics were put into an anaerobic pack (MGC) immediately after they were collected, and then stored at -80°C before initiation of the experimentation (Yang *et al*., 2013). During the experimentation, the stored sample was melted in an anaerobic chamber (Coy laboratory), to which 1:2 (w/v) of sterilized PBS was later added, and homogenized for 2 minutes. The experiment and control groups used for this experiment are as shown in Table 2 below.

**[Table 3]**

| # | **Strains** | **Key feature** |
|---|---|---|
| 1 | PBS | Negative control |
| 2 | CJLS03 | The strain of the present disclosure |
| 3 | OK101 | Granted anti-obesity strain (*L. sakei*) |
| 4 | LGG | Probiotics strain (*L. rhamnosus*) |

5 × 10⁸ CFU of each of the prepared strains according to Table 3 was inoculated in homogenized appendix samples. Sterilized PBS was used as a negative control. The strains inoculated into the appendix sample were shake incubated at 37°C for 24 hours at maximum in an anaerobic environment. During the incubation, sampling of the culture was carried out at 6 hours from each group, and the SCFA of each group was analyzed according to the SCFA analysis method. Bead beating (Biospec, 607) was performed for the appendix contents sampled from the mouse using 600 µL of lysis buffer (Promega ReliaPrep lysis buffer) and 0.3 g of 0.1 mm zirconium/silica beads. Upon completion of the bead beating, the samples were centrifuged, and the supernatant was collected to extract RNA according to the instruction manual of the kit.

The total 20 µL of the extracted RNA in the elute buffer was reverse transcribed to cDNA using Promega GoScript reverse transcription system, and the microbiota in the sample was analyzed using takara RR820 SYBR Master Mix using ABI stepone plus or ABI7500 quantitative real time PCR. The microbiota was analyzed through real-time PCR in a reverse transcribed DNA sample according to the known Gut Low-Density Array (GULDA) method (Bergstrom *et al*., 2012).

Further, the taxa bacteria were quantified using a primer standardized according to the GULDA method, and delta-delta Ct analysis method was drawn based on the quantitative values of universal bacteria and each bacterial control group for comparative quantification.

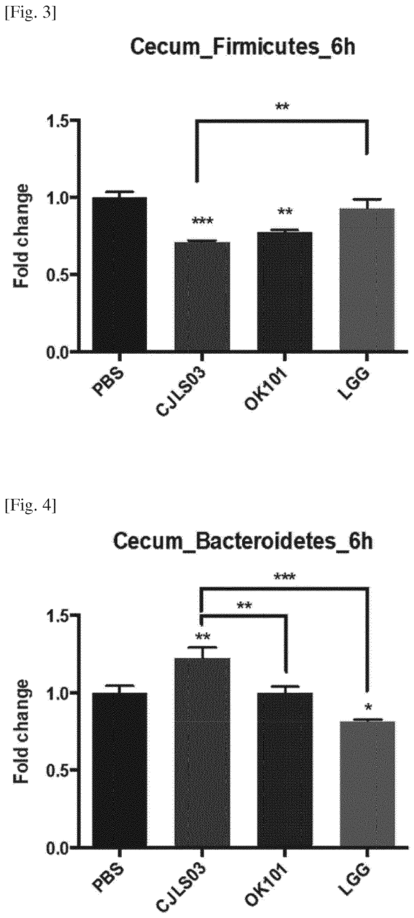

Fig. 3 is a graph showing comparative amounts of *Firmicutes* measured after 6 hours of incubation of each group in a cecum sample, and Fig. 4 is a graph showing comparative amounts of *Bacteroidetes* measured after 6 hours of incubation of each group in a cecum sample.

Based on Figs. 3 and 4, the amount of microorganism of phylum *Firmicutes* in the microbiota was reduced when the *Lactobacillus sakei* CJLS03 of the present disclosure was incubated in the cecum sample for 6 hours, whereas that of phylum *Bacteroidetes* was increased. However, when a conventionally known *Lactobacillus* strain OK101 or LGG was incubated, the amount of microorganism of phylum *Firmicutes* in the microbiota did not decrease or was slightly decreased, and that of phylum *Bacteroidetes* did not increase.

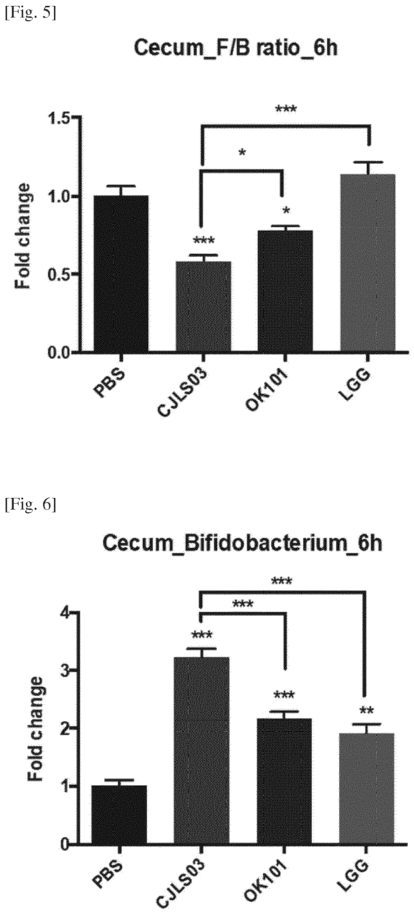

Further, Fig. 5 is a graph showing comparative ratios of *FirmicuteslBacteroidetes* measured after 6-hour incubation of each group in the cecum sample.

Based on Fig. 5, the *FirmicuteslBacteroidetes* ratio of the cecum sample in which the *Lactobacillus sakei* CJLS03 strain was incubated was significantly lower than those in which other *Lactobacillus sakei* strains were applied. That is, the *Lactobacillus sakei* CJLS03 strain of the present disclosure can lower the intestinal *Firmicutes*/*Bacteroidetes* ratio, and thus can have an anti-obesity efficacy and a prophylactic or therapeutic efficacy of metabolic diseases.

Additionally, Fig. 6 is a graph showing comparative amounts of *Bifidobacterium* measured after 6-hour incubation of each group in the cecum sample. The *Bifidobacterium* is another representative beneficial intestinal bacterium like *Lactobacillus.*

Based on Fig. 6, when the *Lactobacillus sakei* CJLS03 was incubated, the amount of the *Bifidobacterium* was significantly increased. That is, compared to previously known strains, the *Lactobacillus sakei* CJLS03 of the present disclosure can exhibit the anti-obesity effect as well as prophylactic and therapeutic effects of metabolic diseases while increasing beneficial intestinal bacteria.

### Example 4: Experiment with obesity induced mice with high fat diet for anti-obesity efficacy of Lactobacillus sakei CJLS03

### 1) Administration method and experimental period

From the time of arrival, all animals underwent a 6-week induction period, including a one-week acclimation period. The mice were divided into normal diet (negative control) and high fat diet (positive control and experiment group) and each fed, while given feed and water *ad libitum* for 6 weeks from the day of division (day 0). The CJLS03 according to the present disclosure was grouped according to concentrations (low: 10⁸ CFU, medium: 10⁹ CFU, high: 10¹⁰ CFU). To measure accurate efficacy, a commercially available FDA-approved anti-obesity drug Orlistat (Product name: Zenical), and a health function food Garcinia cambogia were added as experiment groups in addition to the normal diet control group and high fat diet group, and compared with the anti-obesity function of the lactic acid bacterial. The group details are as shown in Table 4 below.

**[Table 4]**

| **Groups** | | | **Dose** |
|---|---|---|---|
| 1 | ND | Control group having normal diet | - |
| 2 | HFD | Control group having high fat diet | - |
| 3 | OLS | Orlistat (Xenical) | 40 mg/kg |
| 4 | GC | Garcinia | 300 mg/kg |
| 5 | Low CJLS03 | Low concentration of CJLS03 | 1 × 10⁸ CFU |
| 6 | Medium CJLS03 | Medium concentration of CJLS03 | 1 × 10⁹ CFU |
| 7 | High CJLS03 | High concentration of CJLS03 | 1 × 10¹⁰ CFU |

For administration, the lactic acid bacteria were homogeneously suspended in PBS and administered in a 10 mL/kg of administration volume twice daily at determined times (10:00 A.M. to 11:00 A.M., 5:00 P.M. to 6:00 P.M.) for 7 weeks, while the control groups were orally administered with the same amount of solvent vehicle (PBS) twice daily for 7 weeks. Experimental substances were newly prepared and administered into the A.M. and P.M.-administered strain. All strains were stored under refrigeration so as to retain the active condition thereof, and the PBS which was refrigerated was used for washing strain samples and preparing solutions. The experimental animal of each group was fasted for 16 hours and sacrificed on the termination day of experiment (day 49) to be used for blood biochemistry tests and histological evaluation analysis.

### 2) Measurements of body weight and feed intake

To measure the changes of the body weight according to time, the body weight was measured once per week for the obesity-induction period (6 weeks) and twice per week for the experimental substance-administration period (7 weeks) at a predetermined time. The daily feed amount was measured once a week (when measuring the body weight) during the entire experiment period (6 weeks) at a predetermined time by subtracting the amount of the remainder measured after 24 hours from the feed amount.

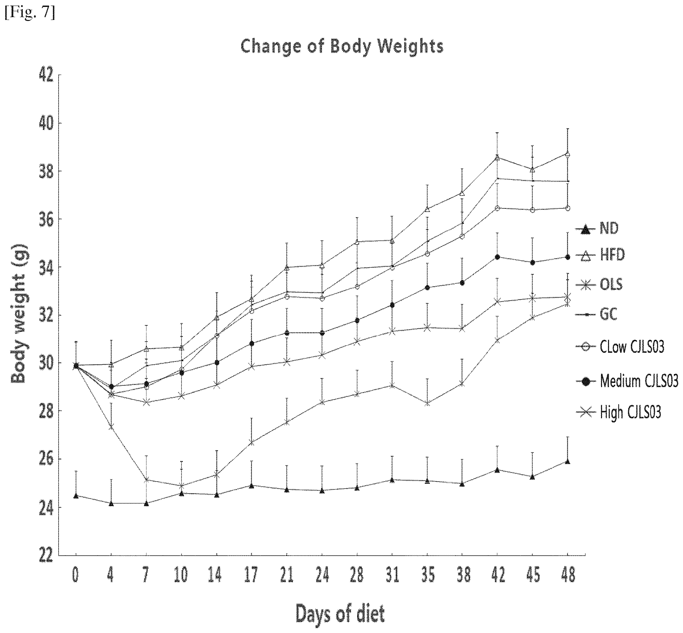

Fig. 7 is a graph showing the body weight changes of each group according to the above Example.

Based on Fig. 7, when the time at which the inhibition effect against body weight increase appears and the degree of the effect were synthetically analyzed, the *Lactobacillus sakei* CJLS03 strain of the present disclosure was confirmed to show a more excellent inhibition effect against body weight increase as its concentration increased. In particular, the inhibition effect against body weight increase of the *Lactobacillus sakei* CJLS03 strain showed not only a higher level of the effect than the anti-obesity health functional food material Garnicia, but also a similar level of the effect to the FDA-approved anti-obesity drug Orlistat.

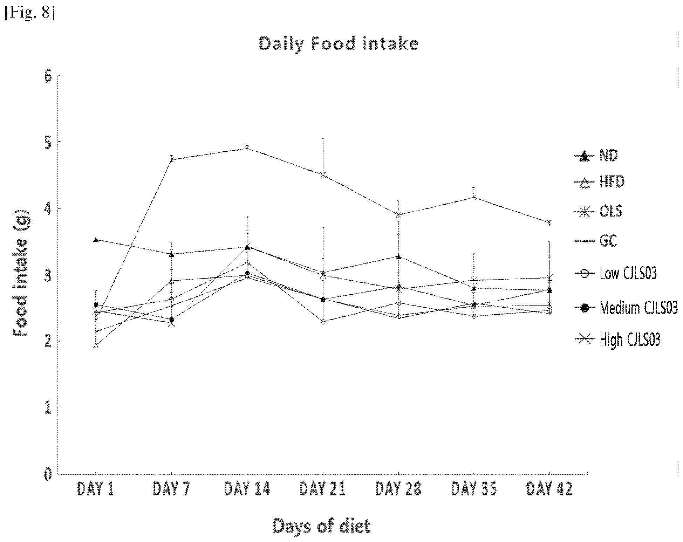

Fig. 8 is a graph showing the amounts of feed intake measured according to the above Example and showing daily food intake measured once per week for 7 weeks.

Based on Fig. 8, the feed intake of all groups excluding the OLS group was overall lower than or similar to that of the ND group, and did not exhibit a great increase or decrease while maintaining a consistent level overall. This indicates that the feed additives excluding Orlistat do not have significant influence on the feed intake of animals.

In light of the above results, the group in which the *Lactobacillus sakei* CJLS03 strain of the present disclosure was administered did not present a significant increase in the body weight compared to the other groups even though it showed a similar amount of feed intake. That is, the *Lactobacillus sakei* CJLS03 strain of the present disclosure was confirmed to have a more excellent anti-obesity effect than known anti-obesity materials and showed a stronger anti-obesity effect as its concentration increased.

### 3) Autopsy and blood biochemistry examination

On the day of termination of the experiment according to Example 4-1 (day 49), the experimental animals were sacrificed after stopping their diets for 16 hours and autopsied. All of the experimental animals were subjected to collection of about 500 µL to 700 µL of blood using a heparin-free capillary tube of plexus venosus under an ether inhalation anesthesia. The collected blood was put in an SST tube and coagulated for 30 minutes to 1 hour at room temperature. The blood was then centrifuged at 4°C and 4000 rpm for 10 minutes to separate the serum followed by storing at -80°C until the analysis. The amounts of glucose (GLU), triglyceride (TG), total cholesterol (TC), low-density lipoprotein cholesterol (LDL), high-density lipoprotein cholesterol (HDL), glutamate oxaloacetate transaminase (GOT; AST), glutamate pyruvate transaminase (GPT; ALT), CREA (creatinine), and lactate dehydrogenase (LDH) in the separated serum were measured using a blood biochemistry (Accute TBA-40FR, Toshiba Medical System Co., Japan).

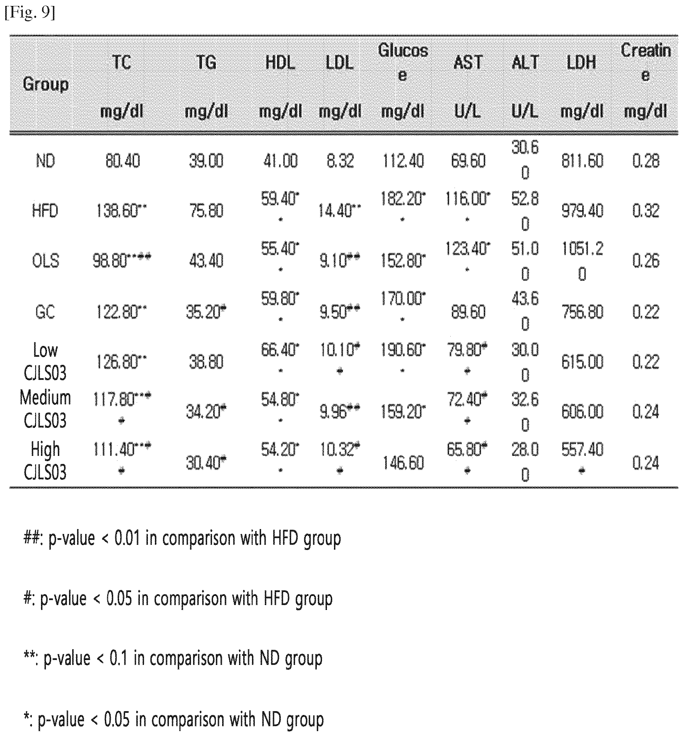

Fig. 9 is a table showing blood components of experimental individuals measured according to the above Example.

Based on Fig. 9, the concentrations of blood glucose, TC, LDL, and HDL measured from the groups provided with a high-fat feed were overall higher than those measured from the ND group. However, those of the OLS or CJKS03 group provided with Orlistat together with the high fat diet were confirmed to significantly reduce compared to those measured in the HFD or GC group. Additionally, blood LDH, ALT, and creatine concentrations, which are indicators of liver, heart, skeletal muscle, or kidney disease, measured from the CJLS03 group, were similar to those measured in the ND group. That is, even when CJLS03 was consumed, it was confirmed that there were no side effects on the functions of kidney, liver, heart, and skeletal muscle. In particular, although it is normal that the concentration of ALT, commonly known as a liver disease index, is 40 u/L or below, the concentration of ALT measured from the OLS group or GC group provided with Orlistat or Garnicia was 40 u/L or above. These were similar to the ALT level measured from the HFD group provided with high fat diet without any additives. However, the ALT level measured from the group provided with CJLS03 was similar to that measured from the ND group that received normal feeds. In other words, it has been confirmed that not only does CJLS03 have an excellent anti-obesity effect, but also, it can prevent or treat liver damage while materials conventionally known to have an anti-obesity effect are likely to cause liver damage or liver disease.

### 4) Measurement of organ fat weight

After blood was collected, the visceral examination was carried out to confirm the presence of abnormalities in the internal organs, and the adipose tissues (epididymal fat, subcutaneous fat, mesenteric fat) were extracted. Each adipose tissue was washed with physiological saline, and the water was removed with a filter paper. The weight of each adipose tissue was measured with an electronic balance. Additionally, the relative tissue weight per 100 g of body weight was calculated based on the body weight fasted before autopsy (calculation formula: relative organ weight (%) = organ weight (g) / fasting body weight before autopsy (g) × 100). The fasted body weight was measured after fasting for 16 hours and was measured before the experimental group was sacrificed.

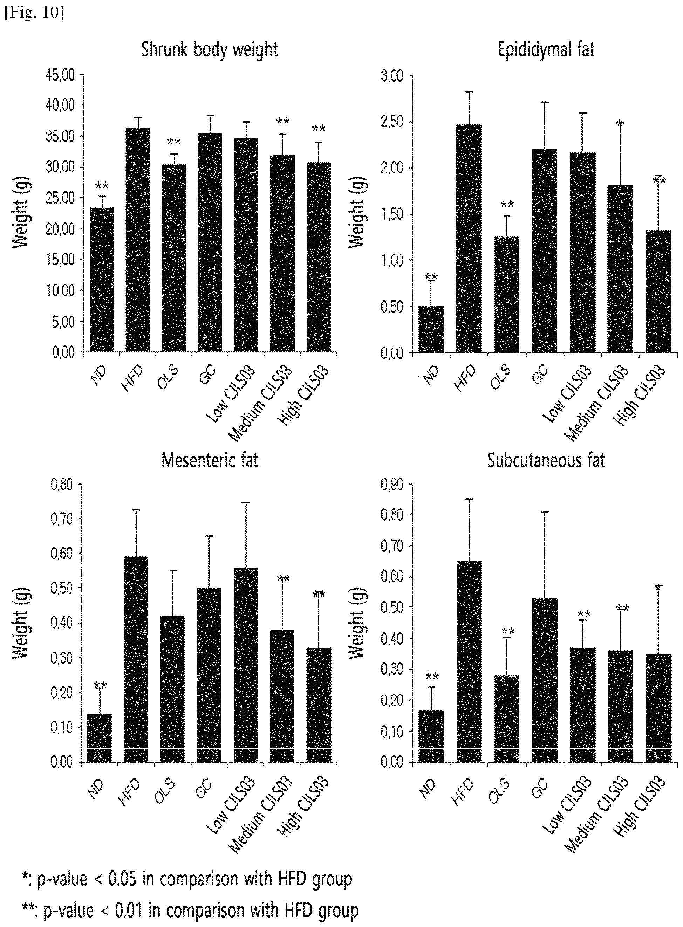

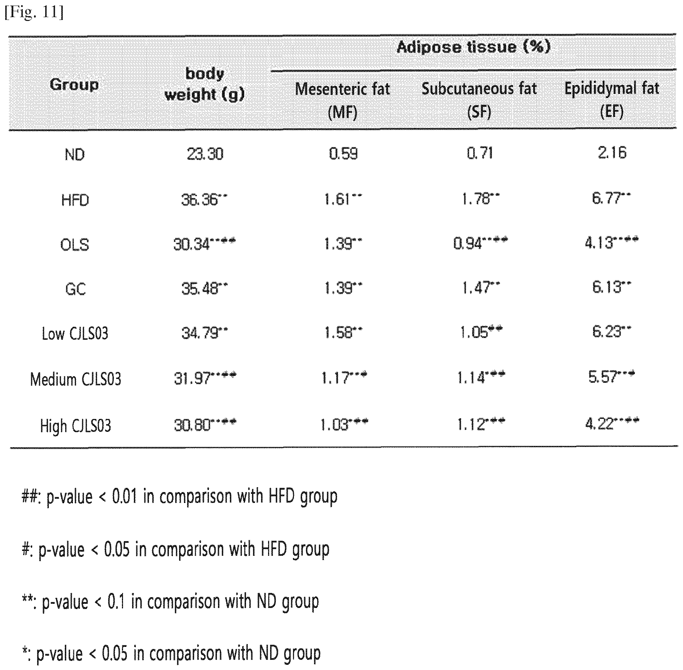

Fig. 10 is a graph showing the weights of body and adipose tissue of each group, and Fig. 11 is a table showing comparative weights of adipose tissue based on the body weight of each group.

Based on Figs. 10 and 11, the fasting and adipose tissue weights of the OLS and CJLS03 groups were lower than those of the HFD group. In particular, the subcutaneous and mesenteric fat weights of the CJLS03 group were significantly reduced compared with the HFD group, and they showed a similar or greater decrease than the OLS group. In other words, the *Lactobacillus sakei* CJLS03 of the present disclosure has an inhibition effect against increase of the body weight and fat, and the anti-obesity efficacy of CJLS03 is similar to or more excellent than Orlistat, a known anti-obesity drug.

### 5) Histological evaluation

Liver tissues of the ND, HFD, OLS, GC, and high CJLS03 concentration groups were histologically evaluated. The liver tissue of each group was embedded, and the paraffin block was dissected with a microtome to a thickness of 4 µm. The paraffin was then removed using xylene, and hydrophilized with 100%, 95%, 90%, 80%, and 70% alcohol, and stained with hematoxylin and eosin (H & E) followed by dehydrating using affiliated alcohol to mount. An optical microscope ((Olympus MVX10 microscope, equipped with a DC71 camera, Center Valley, PA) Olympus, Japan) was used for histological evaluation.

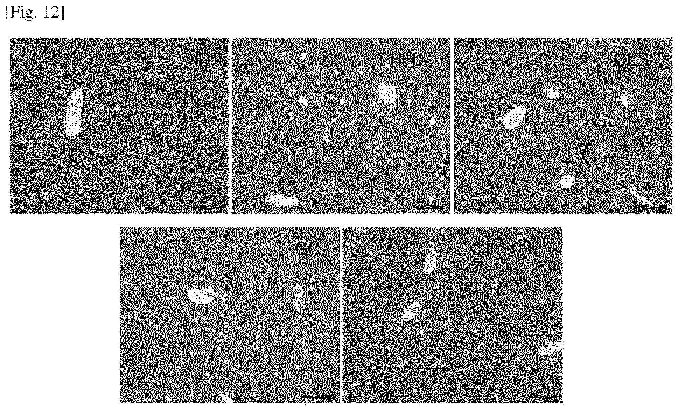

Fig. 12 is photos of histological evaluation of the liver tissues of the ND, HFD, OLS, GC, and high CJLS03 concentration groups.

Based on Fig. 12, white fat particles were found in the liver tissue of the ND group fed with normal diet, whereas the liver tissue of the HFD group with high fat feeds contained many white lipid particles. The liver tissue of the GC group also contained a large number of lipid particles, similar to the liver tissue of the HFD group. In the liver tissues of the OLS and CJLS03 groups, lipid particles were rarely found and the liver tissues were similar to the ND group provided with normal feed. That is, the *Lactobacillus sakei* CJLS03 of the present disclosure inhibits fat accumulation of liver tissues and improves fat liver.

### 6) Measurement of size of adipocytes

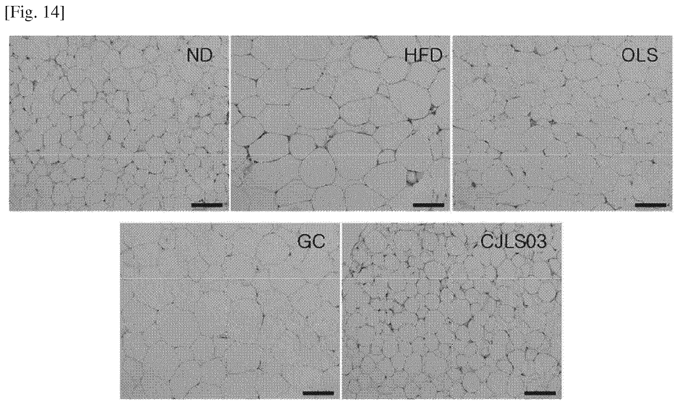

Measuring the size of adipocytes is well known as an effective method capable of proving the anti-obesity efficacy. The photos of adipocytes were obtained from 5 individual tissue slides for each experimental group (CJLS03: high concentration group). Fig. 14 is photos showing the adipocyte size of each group. The adipocyte photos were analyzed using the Scion image program (derived from NIH Image) by calculating an average value of two adipocyte sizes.

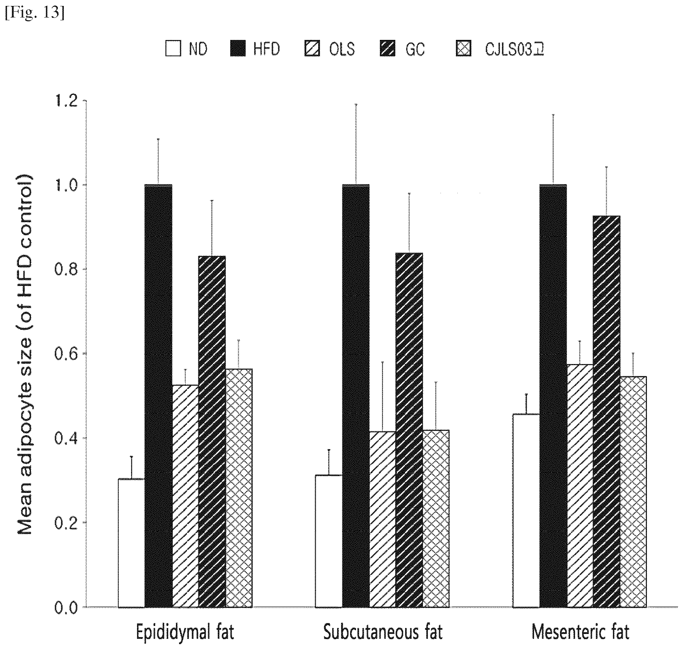

Fig. 13 is a graph showing the size of adipocytes of each group. The size of the adipocytes of each group is presented as a comparative ratio to that of the HFD group.

Based on Fig. 13, the sizes of the adipocytes of the OLS and CJLS03 groups were significantly reduced compared to those of the HFD and GC groups. The size of the adipocytes of the CJLS03 group was reduced to 40% to 70% of those of the HFD group, although they may be somewhat different according to the tissues from which the adipocytes are derived.

In other words, the *Lactobacillus sakei* CJLS03 of the present disclosure decreases not only the fat weight but also size of adipocytes.

### Example 5: Preparation of probiotics comprising Lactobacillus sakei CJLS03

The probiotic *Lactobacillus sakei* CJLS03 identified in Example 1 was mass-produced and lyophilized to be manufactured into probiotics in order to be used as medicine, food, feed, feed additives, or materials for cosmetics.

For strain production, the bacteria were cultured in an MRS liquid medium (Difco) added with 25% NaOH to reach pH 6.0 for 18 hours at 37°C. Then the bacteria were obtained by centrifugation. The collected bacteria were then frozen at -40°C using 5% dextrin and 10% skim milk as a cryoprotectant, and the dried bacteria was ground using a mixer to be in a powder form. The powdered bacteria were mixed with an appropriate amount of a diluting agent such as glucose, lactose, skim milk, *etc.* and stored and packed in an aluminum pouch bag.

The thus-prepared probiotics can be used as feed probiotics by mixing with cereal flour used for raw materials in feeds, or as medicine such as tablets, capsules, *etc.* by mixing carriers or additives, as probiotics for foods. A particular amount of the probiotics may be mixed with raw materials used for cosmetics to be used in various fields such as medicines, foods, feeds, cosmetics, *etc.* according to conventional methods in the art.

From the foregoing, those of ordinary skill in the art will recognize that the present disclosure may be embodied in other specific forms without departing from its spirit or essential characteristics. In this regard, the described embodiments are to be considered in all respects only as illustrative and not restrictive. The scope of the present disclosure is therefore indicated by the appended claims rather than by the foregoing description. All changes which come within the meaning and range of equivalency of the claims are to be embraced within the scope of the present disclosure.

## Claims

1. A *Lactobacillus sakei* CJLS03 strain (KCCM11905P).

2. The *Lactobacillus sakei* CJLS03 strain of claim 1, wherein the strain has activity of promoting intestinal production of a short chain fatty acid or controlling a gut microbiota.

3. The *Lactobacillus sakei* CJLS03 strain of claim 2, wherein the short chain fatty acid is at least one selected from the group consisting of acetate, propionate, and butyrate.

4. The *Lactobacillus sakei* CJLS03 strain of claim 1, wherein the strain has activity of reducing a ratio of intestinal *Firmicutes*/*Bacteroidetes* (F/B).

5. The *Lactobacillus sakei* CJLS03 strain of claim 1, wherein the strain has activity of reducing an aspartate transaminase (AST; GOT) level or an alanine transaminase (ALT; GPT) level.

6. A composition comprising a *Lactobacillus sakei* CJLS03 strain (KCCM11905P).

7. The composition of claim 6, wherein the strain has activity of promoting intestinal production of a short chain fatty acid or controlling a gut microbiota.

8. The composition of claim 7, wherein the short chain fatty acid is at least one selected from the group consisting of acetate, propionate, and butyrate.

9. The composition of claim 6, wherein the strain has activity of reducing a ratio of intestinal *FirmicuteslBacteroidetes* (F/B).

10. The composition of claim 6, wherein the strain has activity of reducing an aspartate transaminase (AST; GOT) level or an alanine transaminase (ALT; GPT) level.

11. The composition of claim 6, wherein the composition further comprises a cryoprotectant or an excipient.

12. The composition of claim 11, wherein the cryoprotectant is at least one selected from the group consisting of glycerol, trehalose, maltodextrin, powdered skim milk, and starch, and the excipient is at least one selected from the group consisting of glucose, dextrin, and nonfat milk.

13. The composition of claim 6, wherein the composition is for prevention, improvement, or treatment of a metabolic disease.

14. The composition of claim 13, wherein the metabolic disease is selected from the group consisting of obesity, diabetes, hypertension, hyperlipidemia, arteriosclerosis, stroke, and a cardiovascular disorder.

15. The composition of claim 6, wherein the composition is a food, a functional food, a feed, a feed additive, a cosmetic composition, a pharmaceutical composition, or a quasi-drug.


<110>     CJ CheilJedang Corporation

<120>     Novel Lactobacillus sakei and compositions comprising the same

<130>     OPA17141-PCT

<150>     10-2016-0126823
<151>     2016-09-30

<160>     1

<170>     KoPatentIn 3.0

<210>     1
<211>     1466
<212>     RNA
<213>     Unknown

<220>
<223>     Lactobacilluse sakei CJLS03


<400>     1
gggngtgcta tantgcagtc gaacgcactc tcgtttagat tgaaggagct tgctcctgat        60

tgataaacat ttgagtgagt ggcggacggg tgagtaacac gtgggtaacc tgccctaaag       120

tgggggataa catttggaaa cagatgctaa taccgcataa aacctaacac cgcatggtgt       180

agggttgaaa gatggtttcg gctatcactt taggatggac ccgcggtgca ttagttagtt       240

ggtgaggtaa aggctcacca agaccgtgat gcatrgccgw sckgrgaggg taatcggcca       300

cactgggact gagacacggc ccagactcct acgggaggca gcagtaggga atcttccaca       360

atggacgaaa gtctgatgga gcaacgccgc gtgagtgaag aaggttttcg gatcgtaaaa       420

ctctgttgtt ggagaagaat gtatctgata gtaactgatc aggtagtgac ggtatccaac       480

cagaaagcca cggctaacta cgtgccagca gccgcggtaa tacgtaggtg gcaagcgttg       540

tccggattta ttgggcgtaa agcgagcgca ggcggtttct taagtctgat gtgaaagcct       600

tcggctcaac cgaagaagtg catcggaaac tgggaaactt gagtgcagaa gaggacagtg       660

gaactccatg tgtagcggtg aaatgcgtag atatatggaa gaacaccagt ggcgaaggcg       720

gctgtctggt ctgtaactga cgctgaggct cgaaagcatg ggtagcaaac aggattagat       780

accctggtag tccatgccgt amacgatgag tgctaggtgt tggagggttt ccgcccttca       840

gtgccgcagc taacgcatta agcactccgc ctggggagta cgaccgcaag gttgaaactc       900

aaaggaattg acgggggccc gcacaagcgg tggagcatgt ggtttaattc gaagcaacgc       960

gaagaacctt accaggtctt gacatccttt gaccactcta gagatagagc tttcccttcg      1020

gggacaaagt gacaggtggt gcatggttgt cgtcagctcg tgtcgtgaga tgttgggtta      1080

agtcccssma cgagcgcaac ccttattact agttgccagc atttagttgg gcactctagt      1140

gagactgccg gtgacaaacc ggaggaaggt ggggacgacg tcaaatcatc atgcccctta      1200

tgacctgggc tacacacgtg ctacaatgga tggtacaacg agttgcgaga ccgcgaggtt 1260

tagctaatct cttaaaacca ttctcagttc ggattgtagg ctgcaactcg cctacatgaa 1320

gccggaatcg ctagtaatcg cggatcagca tgccgcggtg aatacgttcc cgggccttgt 1380

acacaccgcc cgtcacacca tgagagtttg taacacccaa agccggtgag gtaacccttc 1440

ggggagccag ccgtctaang nggaca 1466